(19) Europäisches Patentamt
European Patent Office
Office européen des brevets

(11) Publication number: **0 355 085 B1**

(12) **EUROPEAN PATENT SPECIFICATION**

(45) Date of publication of patent specification: **11.09.91 Bulletin 91/37**

(51) Int. Cl.$^5$: **C07D 473/30**

(21) Application number: **88901029.4**

(22) Date of filing: **04.01.88**

(86) International application number: **PCT/EP88/00001**

(87) International publication number: **WO 88/05437 28.07.88 Gazette 88/17**

# (54) A PROCESS FOR THE PREPARATION OF 9-(HYDROXYALKYL)-HYPOXANTHINES.

(30) Priority: **14.01.87 IT 1908387**

(43) Date of publication of application: **28.02.90 Bulletin 90/09**

(45) Publication of the grant of the patent: **11.09.91 Bulletin 91/37**

(84) Designated Contracting States: **AT BE CH DE FR GB IT LI LU NL SE**

(56) References cited:
**EP-A- 0 009 154**
**EP-A- 0 009 155**
**JOURNAL OF MEDICAL CHEMISTRY, vol. 8, no.4, July 1965 (Columbus, Ohio, US), H.J. Schaeffer et al.: "Enzyme inhibitors: VIII. Studies on the mode of binding of some 6-substituted 9-(hydroxyalkyl)purines to adenosine deaminase", pages 502-506**
**JOURNAL OF MEDICAL CHEMISTRY, vol. 10, no. 4, July 1967 (Columbus, Ohio, US), H.J. Schaeffer et al.: "Enzyme inhibitors. XVIII. Studies on the stereoselectivity of inhibition of adenosine deaminase by DL-, D-, and L-9-(2-hydroxypropyl)adenine", pages 689-691**

(73) Proprietor: **CO PHARMA CORPORATION S.R.L.**
**Via Assarotti, 52**
**I-16123 Genova (IT)**

(72) Inventor: **STRADI, Riccardo**
**Via Sansovino, 33**
**I-20133 Milan (IT)**

(74) Representative: **Bianchetti, Giuseppe**
**Studio Consulenza Brevettuale Via Rossini, 8**
**I-20122 Milan (IT)**

**EP 0 355 085 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an novel and improved process for the preparation of 9-(hydroxyalkyl)-hypoxanthines of general formula I

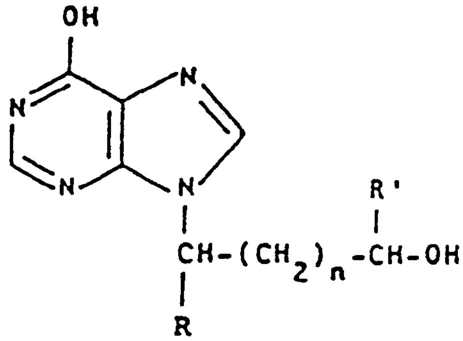

(I)

In formula I above, R is hydrogen or a straight or branched $C_1$-$C_{10}$ alkyl group ; R′ is hydrogen or a straight or branched $C_1$-$C_4$ alkyl group ; n is an integer 0 to 5.

Compounds (I) are important intermediates for the synthesis of compounds having antiviral, antitumoral and immunomodulating activities as described, for example, in EP-A-9155.

A suitable process for obtaining the compounds of general formula (I) is already known both in scientific and patent literature. For example, in J. Chem., 8, 502 (1965) and in J. Med. Chem., 10, 689 (1967) synthesis of compounds (I) is described, according to the following scheme :

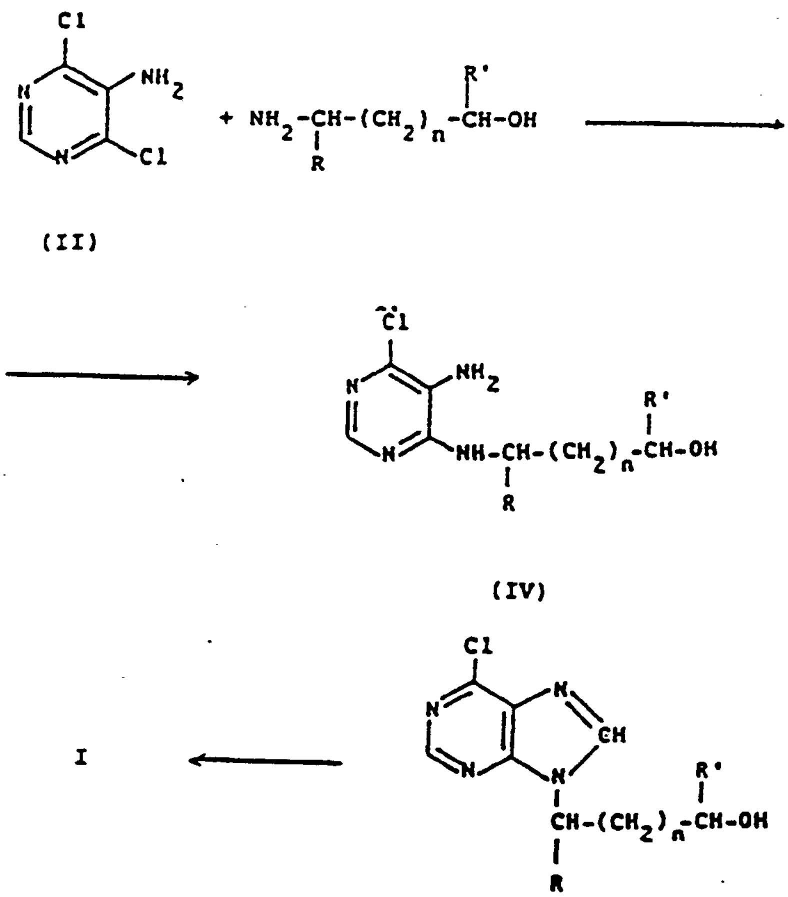

(II)

(IV)

I

(V)

According to said process, 4,6-dichloro-5-amino-pyrimidine (II) is reacted with an aminoalcohol of formula (III) to give the corresponding 5-amino-6-chloro-4-(hydroxyalkylamino)-pyrimidine (IV) which is cyclized to 6-chloro-9-(hydroxyalkyl)-purine (V) with an excess of ethyl orthoformate and compound (V) is finally hydrolyzed

to corresponding compound (I).

The main drawback of said reaction resides in the use of a large excess of ethyl orthoformate, which is known to be remarkably expensive and hardly available on industrial scale, as well as in the practical difficulties connected with the purification of the intermediate purine derivative (V). This applies particularly when n is an integer above 2.

Now, it has been surprisingly found that compounds of general formula (I), wherein R, R' and n have the above mentioned meanings, may be advantageously obtained by an economic process, when the above defined compound IV is cyclized with formic acid instead of ethyl orthoformate.

Thus, the present invention relates to a process for the preparation of 9-(hydroxyalkyl)-hypoxanthines of general formula (I), wherein R, R' and n have the above mentioned meanings, in which process a 5-amino-6-chloro-4-(hydroxyalkylamino)-pyrimidine of general formula (IV)

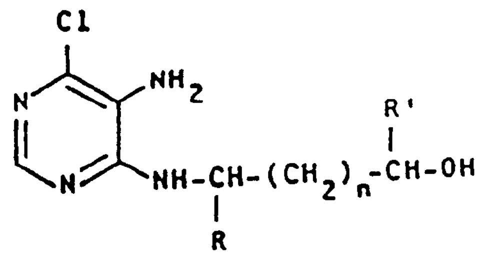

(IV)

wherein R, R' and n have the above mentioned meanings, in form of the hydrochloride or of other salts, is refluxed with concentrated formic acid (HCOOH), to give the compound of general formula (I).

The process of the invention is carried out by heating to the reflux temperature a solution or suspension of compound (IV), as the hydrochloride or another salt, in formic acid of suitably high concentration, preferably 99%.

The reaction is carried out for a time ranging from 10 to 20 hours, suitably 15 hours. The highly pure final compound is recovered according to usual techniques.

The scheme hereinbelow summarizes the process of the invention :

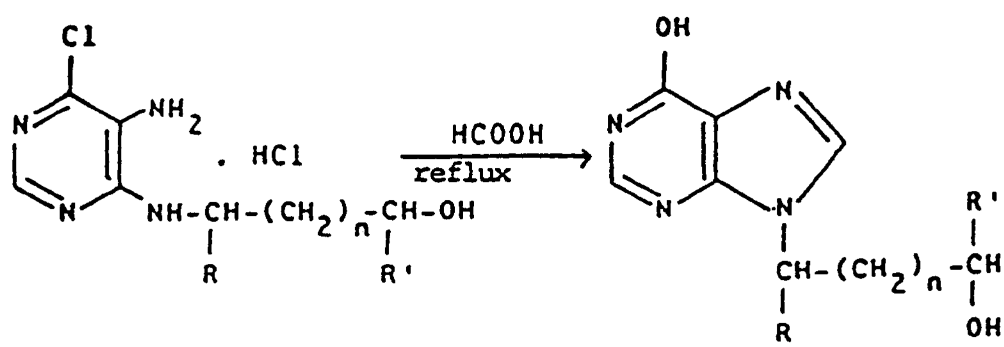


(IV) (I)

The process of the invention has remarkable advantages. First, use of expensive ethyl orthoformate, which is replaced by much more economic formic acid, is avoided. On the other hand, treatment of derivative (IV) according to the invention carries out in a single operative step both cyclization, with formation of purine ring, and hydrolysis of chlorine substituent, thus avoiding the need for isolation of intermediate (V) with evident savings in time, labour and materials.

The following examples further illustrate the invention.

<u>**EXAMPLE 1**</u>

<u>**9-(4-hydroxybutyl)-hypoxanthine (Formula I, R = H ; R' = H ; n = 2)**</u>

16.4 g (0.1 mole) of 4,6-dichloro-5-aminopyrimidine, 18.5 g (0.1 mole) of tributylamine and 8.91 g (0.1 mole)

of 4-amino-1-butanol were placed into a 250 ml flask.

The mixture was heated to reflux for 15 hours, cooled and treated with a gaseous HCl flow to persistent acidity, then cooled, left to stand at 0°C for 12 hours. 4-chloro-5-amino-6-(4-hydroxybutyl)-amino-pyrimidine hydrochloride was filtered and dried at 60°C.

The crude dried hydrochloride (22,2 g) was dissolved in 99% formic acid and refluxed for 15 hours. Solvent was evaporated off, the residue was dissolved in the minimun water amount, treated with $NH_3$ at 10-20°C for some hours, and the white precipitate formed was filtered and recrystallized from ethanol, to yield 18.2 g. Melting point 200-202°C.

## EXAMPLES 2-9

Following an analogous process, using corresponding amounts of the appropriate aminoalcohol, the compounds listed in the following table were obtained :

**Examples 2-9 : compounds of general formula I**

| Examples | R | R' | n | m.p. (°C) |
|---|---|---|---|---|
| 2 | H | H | 0 | 277-280 |
| 3 | H | H | 1 | 250-254 |
| 4 | H | H | 3 | 190-192 |
| 5 | H | H | 4 | 151-153 |
| 6 | H | $CH_3$ | 0 | 244-248 |
| 7 | $CH_3$ | H | 0 | 206-208 |
| 8 | $C_6H_{13}$ | $CH_3$ | 0 | 196-198 |
| 9 | $C_2H_5$ | H | 0 | 198-200 |

## Claims

1. A process for the preparation of 9-(hydroxyalkyl)-hypoxanthines of general formula I

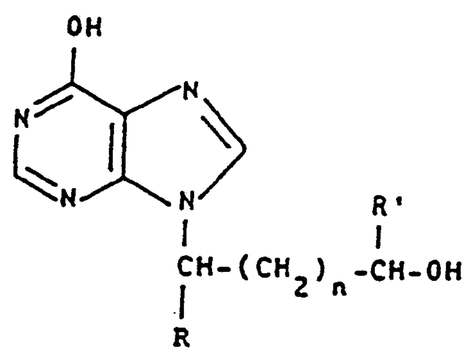

(I)

wherein R is hydrogen or a straight or branched $C_1$-$C_{10}$ alkyl group ; R' is hydrogen or a straight or branched $C_1$-$C_4$ alkyl group ; n is an integer 0 to 5, in which process a 5-amino-6-chloro-4-(hydroxylalkylamino)-pyrimidine of general formula (IV)

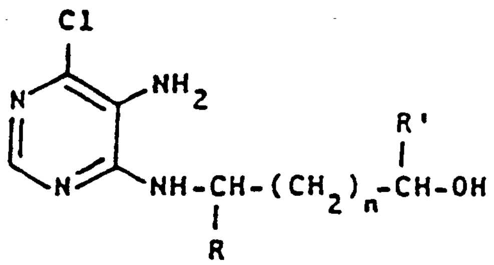

(IV)

wherein R, R' and n have the above mentioned meanings, is reacted with formic acid.

2. A process according to claim 1, wherein compound (IV) is in form of the hydrochloride or of another salt.

3. A process according to claims 1 and 2, wherein formic acid is 99% formic acid.

4. A process according to claims 1-3, wherein the reaction is carried out at the reflux temperature, for a time ranging from 10 to 20 hours.

## Patentansprüche

1. Verfahren zur Herstellung von 9-(Hydroxyalkyl)-hypoxanthinen der allgemeinen Formel I

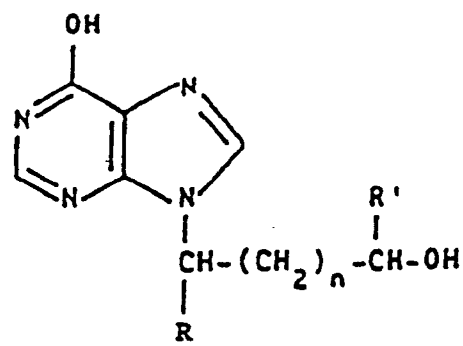

(I)

worin R Wasserstoff oder eine geradkettige oder verzweigte $C_1$-$C_{10}$ Alkylgruppe ist; R' Wasserstoff oder eine geradkettige oder verzweigte $C_1$-$C_4$ Alkylgruppe ist; n eine ganze Zahl von 0 bis 5 ist, gemäß dem ein 5-Amino-6-chlor-4-(hydroxylalkylamino)-pyrimidin der allgemeinen Formel (IV)

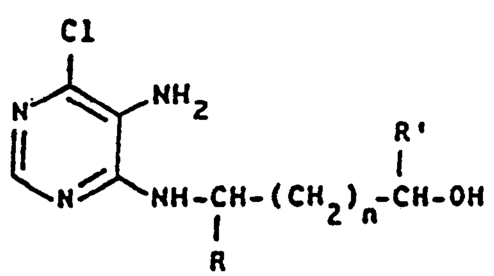

(IV)

worin R, R' und n die gleiche Bedeutung wie oben haben, mit Ameisensäure umgesetzt wird.

2. Verfahren gemäß Anspruch 1, wobei die Verbindung (IV) in Form des Hydrochlorides oder eines anderen Salzes vorliegt.

3. Verfahren gemäß Anspruch 1 und 2, worin die Ameisensäure 99%ige Ameisensäure ist.

4. Verfahren gemäß Ansprüchen 1-3, worin die Reaktion unter Rückflußtemperatur für einen Zeitraum von 10 bis 20 Stunden durchgeführt wird.

## Revendications

1. Procédé de fabrication d'(hydroxyalkyl)-9 hypoxanthines de formule générale (I) :

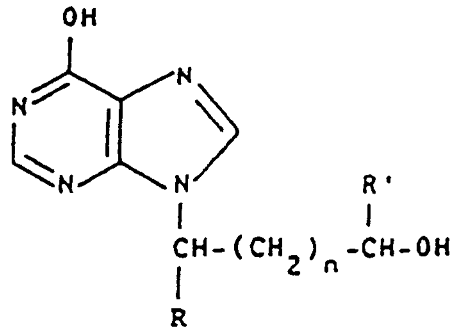

(I)

dans laquelle R représente hydrogène ou un groupe alkyle, à chaîne droite ou ramifiée, en $C_1$-$C_{10}$ ; R' représente hydrogène ou un groupe alkyle, à chaîne droite ou ramifiée, en $C_1$-$C_4$ ; et n est un nombre entier de 0 à 5, procédé suivant lequel on fait réagir avec l'acide formique une amino-5 chloro-6 (hydroxyalkylamino)-4 pyrimidine de formule générale (IV) :

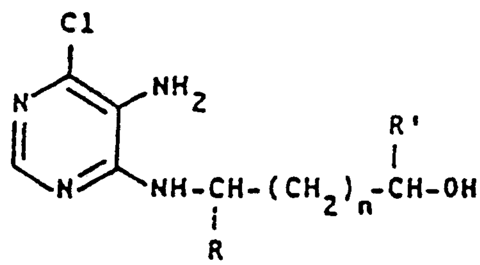

(IV)

dans laquelle R, R' et n ont les mêmes significations que celles mentionnées ci-dessus.

2. Procédé selon la revendication 1, dans lequel le composé (IV) se présente sous la forme du chlorhydrate ou d'un autre sel.

3. Procédé selon les revendications 1 et 2, dans lequel l'acide formique est de l'acide formique à 99%.

4. Procédé selon les revendications 1 à 3, dans lequel la réaction est effectuée à la température de reflux, pendant une période de temps de 10 à 20 heures.